# EUROPEAN PATENT APPLICATION

(11) **EP 2 666 365 A1**
(43) Date of publication of application: **27.11.2013**
(21) Application number: 12168934.3
(22) Date of filing: 22.05.2012
(51) Int. Cl.: A23K 1/16

(54) **Treatment of poultry, pigs or fish for reducing the feed conversion ratio.**

(71) Applicant: Taminco, 9000 Gent (BE)
(72) Inventor: Lauwaerts, Angelo, 9000 Gent (BE); Laget, Mia, 9220 Hamme (BE); de Moor, Camille, 9050 Gentbrugge (BE)
(74) Representative: Van Reet, Joseph

(57) **Abstract**

The invention relates to a method for the non-therapeutic treatment of animals selected from the group consisting of poultry, pigs and fish. The treatment comprises orally administering at least one ß-alanine compound to the animals in an amount of between 2 and 55 mmol/kg dry weight of said feed for the treatment of poultry and fish and between 2 and 25 mmol/kg dry weight of said feed for the treatment of pigs. These amounts of the ß-alanine compound are used for reducing the conversion ratio or for increasing the bodyweight gain of the animals. The ß-alanine compound is in particular ß-alanine. In prior art methods higher amounts were used resulting also in a reduction of the feed conversion ratio but this to the detriment of the bodyweight gain.

## Description

The present invention relates to a method for the non-therapeutic treatment of animals selected from the group consisting of poultry, pigs and fish for the purpose of reducing the conversion ratio of the feed used to feed the animals without lowering their bodyweight gain.

In the meat producing industry, improvements and developments have been made essentially in the breeding technique for phyletic lines of the animals and in the rearing technique for increasing the bodyweight gain thereof. This is especially the case in the broiler and in the pork industry (both for grower and finisher pigs) but also in the fish industry. Much emphasis is put on the bodyweight gain of the meat producing animals and the conversion ratio of the feed used to rear them. A high-calorie feed enables to achieve a lower feed conversion ratio, in particular a lower amount of feed is required to produce a certain amount of animal meat or other production parameters such as litres of milk for dairy, total egg weight for layers or total litter weight for reproduction sows. However, a further reduction of the feed conversion ratio is always desired to reduce the production costs. When lowering the feed conversion ratio it is important that the bodyweight gain is not reduced by the applied treatment.

In practice, it is indeed of high economical importance to be able to decrease the feed conversion ratio, i.e. the amount of feed required for 1 kg of productivity, being either gain in bodyweight or, in case of sows, production of weaned piglets, without having to use a (more expensive) feed having a higher energy or nutrient value.

Several additives have already been tried to lower the feed conversion ratio and increase the bodyweight gain of animals.

WO 2007/107184 and WO 2009/033502 disclose for example the use of dimethyl glycine (DMG) for lowering the feed conversion ratio and increasing the bodyweight gain of respectively pigs and broilers.

Other additives which have been tested are carnosine (ß-alanyl-L-histidine) and ß-alanine. Carnosine, and its derivative anserine (ß-alanyl-1-methyl-L-histidine) are known to function as anti-oxidants and putative neurotransmitters. They may thus influence the brain functions and also the meat quality. Hu et al. (2009) have tested the effect of carnosine on growth performance, carcass characteristics, meat quality and oxidative stability in broiler chickens. Supplementation of the broiler feed with 0.5% of carnosine enabled to improve the chicken meat quantity and quality. Both the weight gain and the feed conversion ratio were improved but not statistically significantly.

Carnosine is a dipeptide made up of ß-alanine and histidine. Several prior art publications disclose that it is possible to increase the carnosine level in different tissues by administering ß-alanine instead of carnosine itself to the animal. Since ß-alanine is more easy to produce industrially than carnosine, or is in other words much cheaper, it is thus advantageous to use ß-alanine instead of carnosine.

Tomonaga et al. (2005) have demonstrated that orally administerd ß-alanine increases carnosine concentrations in both the breast muscle and the brains of chickens. They administered 22 mmol/kg bodyweight twice a day for five days to one day old chickens. Calculated based on the feed consumption of the chickens, this amount corresponded on average to about 21000 mg/kg of the feed (wet weight) consumed by the chickens. A drawback of such ß-alanine administration was that, although the feed conversion ratio decreased, the feed consumption and the bodyweight gain also decreased. These results were consistent with the results obtained by Jacob et al. (1991), who supplemented the diet of one day old broiler chickens with 2.5 and 5.0% of ß-alanine. Such high ß-alanine supplementations are therefore not interesting from a commercial point of view. Moreover, although the carnosine level increased in the experiments performed by Tomonaga et al. (2005), the anserine level decreased so that there was no significant increase of the dipeptide (carnosine and anserine) level. Since anserine has even a stronger anti-oxidant activity than carnosine, no effective effects might be obtained by the ß-alanine treatment from the point of view of anti-oxidant activity. Finally, due to the fact that ß-alanine is an antagonist of taurine (i.e. is a taurine transporter inhibitor), the taurine concentration was found by Tomonaga et al. to be significantly decreased (i.e. was reduced with more than 50%) in the breast muscles.

In their next publication of 2006, they tried to increase the sum of dipeptides in the muscles by using more moderate ß-alanine treatments in chickens. More particularly, they supplemented the feed of 24 days old broiler chickens for 4 weeks with 0.5, 1 and 2% of ß-alanine. Carnosine and anserine concentrations in the breast muscles of the chickens were not influenced by this dietary treatment. The ß-alanine concentration however significantly increased whilst the taurine concentration significantly decreased. For the higher concentrations of 1 and 2%, the growth performance parameters were negatively affected, namely the bodyweight gain still decreased significantly whilst the feed conversion ratio now increased instead of decreased. For the lowest concentration of 0.5% no significant changes of the bodyweight gain and of the feed conversion ratio were observed.

From the publications of Tomonaga et al. it thus appears that it would not be possible to reduce the feed conversion ratio in broiler chickens substantially without also reducing the bodyweight gain.

ß-alanine was also supplemented to pigs, namely by Mei et al. (1998) to determine the influence thereof on the oxidative stability of pork. They found out that a supplementation of the pig diet with 0.225% of ß-alanine is not an efficient method to increase the oxidative stability of pork. As to the production parameters, the feed conversion ratio was decreased somewhat by the ß-alanine supplementation but the bodyweight gain also decreased somewhat. In combination with histidine, the bodyweight gain decreased even more and the feed conversion ratio increased to become higher than the control. Mei et al. therefore doesn't teach that it is possible to decrease the feed conversion ratio by means of a ß-alanine compound without reducing the bodyweight gain.

Supplementation of the diet of fish, more particularly of Japanese flounders, with 8.9 g/kg of ß-alanine was done by Kim et al. (2003). The bodyweight gain was increased somewhat but not significantly. The feed conversion ratio (incorrectly designated as feed efficiency by Kim et al.) was however increased significantly by the ß-alanine supplementation. This feed conversion ratio, ranging between 1.43 and 1.76, was incorrectly designed as feed efficiency by Kim et al. (as a matter of fact, a feed efficiency measured based on the bodyweight gain cannot be larger than 1). The feed efficiency is indeed the inverse of the feed conversion ratio and thus ranges in the results of Kim et al. between 0.57 and 0.7. This is consistent with the feed efficiency of flounder determined with a same food (protein-to-lipid level which was also 50:10%) by Hebb et al. (2003) to be equal to 0.7.

An object of the present invention is to provide an alternative non-therapeutic treatment of poultry, pigs or fish which enables to reduce the conversion ratio of the feed used to feed these animals without reducing however the bodyweight gain, i.e. the average weight gain, or which even enables to increase the bodyweight gain.

In a first aspect, the present invention concerns a method for the non-therapeutic treatment of animals selected from the group consisting of poultry, pigs and fish, which treatment comprises orally administering at least one ß-alanine compound to the animals in an amount of between 2 and 55 mmol/kg dry weight of said feed for the treatment of poultry and fish and between 2 and 25 mmol/kg dry weight of said feed for the treatment of pigs, which ß-alanine compound corresponds to the following formula (I): 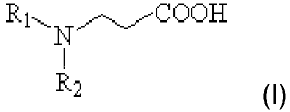 or to a salt or an amide thereof, the amide being of the following formula (II): 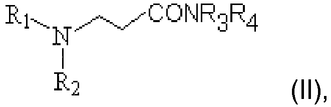 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

In a second aspect, the present invention concerns the use of a ß-alanine compound for reducing the conversion ratio of feed used to feed animals selected from the group consisting of poultry, pigs and fish, without lowering their bodyweight gain, or for increasing the bodyweight gain of the animals, which ß-alanine compound is orally administered to said animals in an amount of between 2 and 55 mmol/kg dry weight of said feed for the treatment of poultry and fish and between 2 and 25 mmol/kg dry weight of said feed for the treatment of pigs, which ß-alanine compound corresponds to the following formula (I): 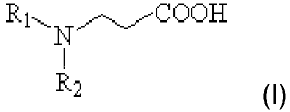 or to a salt or an amide thereof, the amide being of the following formula (II): 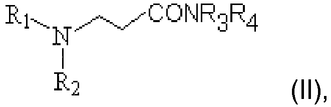 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

In a third aspect, the present invention concerns a feed for poultry or fish comprising between 2 and 55 mmol/kg dry weight of a ß-alanine compound, or a feed for pigs comprising between 2 and 25 mmol/kg dry weight of said ß-alanine compound, which ß-alanine compound corresponds to the following formula (I): 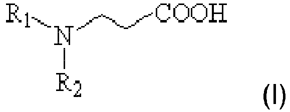 or to a salt or an amide thereof, the amide being of the following formula (II): 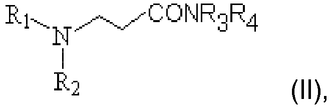 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

In a preferred embodiment, the ß-alanine compound is ß-alanine, N,N-dimethyl ß-alanine, N,N-diethyl ß-alanine, , N,N-di-n-propyl ß-alanine, N,N-diisopropyl ß-alanine, N,N-di-n-butyl ß-alanine, N,N-diisobutyl ß-alanine, N,N-di-tert-butyl ß-alanine, 3-acetamidopropanoic acid or mixtures or salts thereof, for example a sodium, potassium, magnesium or calcium salt, the ß-alanine compound being preferably ß-alanine or a salt thereof.

A difference with the prior art cited here above is that the reduction of the feed conversion ratio is obtained with amounts of the ß-alanine compound which are smaller than those used in the prior art to achieve a reduction of the feed conversion ratio, namely with amounts of the ß-alanine compound which are comprised between 2 and 55 mmol/kg dry weight of the feed, when used for the treatment of poultry or fish, or between 2 and 25 mmol/kg dry weight of the feed, when used for the treatment of pigs. Such small amounts of the ß-alanine compound do not reduce the bodyweight gain but surprisingly still enable to reduce the feed conversion ratio. Moreover, the smaller the amount of the ß-alanine compound, the smaller the cost for the supplementation of the feed with this feed additive.

In a preferred embodiment of the invention, the preferred amount of the ß-alanine compound in the finished feed is at least 5, preferably at least 10 and more preferably at least 15 mmol/kg dry weight of said feed. The maximum amount of the ß-alanine compound in the finished feed is preferably less than 50, preferably less than 40 and more preferably less than 30 and most preferably less than 25 or even less than 20 mmol/kg dry weight of said feed, when used for the treatment of poultry or fish, or preferably less than 22, more preferably less than 20 and most preferably less than 17 mmol/kg dry weight of said feed, when used for the treatment of pigs.

The present invention is applicable to any type of commercial meat production operation. The animals are poultry (i.e. chickens or turkeys), pigs or fish. In commercial pig and poultry production operation the herd is typically under substantial stress. As is well known, normal industry growing conditions include substantial density in the enclosure. Further, the ventilation in such commercial growing operations is often not a precisely controlled operation and the determination of appropriate ventilation including both heating and cooling is a very subjective operation. For broilers, the life span moreover ranges from about 35 to about 49 days whilst the lifespan for turkeys ranges from 12 to 24 weeks. The life span for slaughter pigs is around 6 months whilst sows are usually removed after 3 rounds. Both for poultry and for pigs the whole operation from birth to market in conditions under which growth/reproduction is achieved is therefore very stressful. Moreover, to aggravate the problem, growers will typically push the limits of recommended industry conditions which simply increases the stress on the flock or herd.

Due to these high performance conditions, the occurrence level of metabolic problems, is already quite high in practice and limits the development of new feeds or production methods which causes even more metabolic or oxidative stress. A higher oxidative stress is for example obtained when the feed compositions contain more unsaturated fatty acids, for example more than 2 % by dry weight or more than 3 or even or even more than 4 % by dry weight of the feed, whilst a higher metabolic stress is obtained when the animals are made to take up more calories to increase performance. The fatty acids contained in the feed compositions are either free fatty acids or fatty acids bound for example in di- or triglycerides.

Nowadays animal feed compositions are more and more supplemented with fats from vegetal sources, as a cheaper and more sustainable alternative to fish oil and a safer alternative to animal fat. Hence, because of growing customer demand for use of vegetarian diets in meat production in order to avoid potential hazards peculiar to animal by-products such as PCB, dioxin or BSE contamination. A second reason is to increase the amount of PUFA's (poly unsaturated fatty acids) in the meat, improving the nutritional value of the meat without compromising the total energy value of the feed for the animals. As a direct effect of this increased level of vegetal fat in the feed the dietary induced oxidative stress increases, which leads to genotoxicity (DNA damage) and tissue damage.

The ß-alanine compound administered to the animals is preferably ß-alanine, N,N-dimethyl ß-alanine, N,N-diethyl ß-alanine, N,N-di-n-propyl ß-alanine, N,N-diisopropyl ß-alanine, N,N-di-n-butyl ß-alanine, N,N-diisobutyl I ß-alanine, N,N-di-tert-butyl ß-alanine, 3-acetamidopropanoic acid or a salt of these compounds, for example a sodium, potassium, magnesium or calcium salt. The most preferred ß-alanine compound is ß-alanine or a salt thereof.

When the ß-alanine compound is water-soluble, such as ß-alanine itself, it can be dosed in the drinking water of the animals. Most preferably, the ß-alanine compound is however administered via the feed. The ß-alanine compound can either be added directly to the feed, or to a feed supplement, in particular a so-called premix, which is usually used to prepare the feed. Such a feed supplement generally comprises at least vitamins and minerals.

The ß-alanine compound is preferably administered over a period of 7 days or longer, preferably over a period of 14 days or longer.

### Experimental results

### Poultry:

### Materials and methods

A group of 252 Ross 308 chickens were randomly distributed over 14 pens with 18 animals each. All chickens were previously housed in identical conditions, and fed the same diet as the control group in the trial. Every other pen was attributed to either a control diet or that control diet supplemented with 500 mg ß-alanine per kg (= 595 mg or 6.7 mmol ß-alanine per kg dry weight). Water was freely available from drinking cups, and animals were fed ad libitum. The control diet was a commercial broiler diet (Vanden Avenne, Braadkip 114MB) with 4% corn oil added to increase the level of oxidative stress, as used in earlier studies (Kalmar et al., 2011). The composition of this diet is summarized in Tables 1 and 2.

**Table 1: Ingredient composition of the experimental diet.**

| ***Ingredient*** | **Content, g/kg** |
|---|---|
| | |
| *Wheat* | 538 |
| *Corn* | 29 |
| *Corn oil* | 40 |
| *Toasted soybean meal* | 228 |
| *Toasted soybeans* | 29 |
| *Peas* | 19 |
| *Alphalpha meal* | 10 |
| *Animal fat* | 58 |
| *Soy oil* | 17 |
| *Dicalcium phosphate* | 10 |
| *Limestone* | 8 |
| *Sodium bicarbonate* | 1.2 |
| *Sodium chloride* | 1.7 |
| *Premix** | 4.8 |
| *L-lysine HCl* | 3.4 |
| *DL-methionine* | 3.1 |
| *L-threonine* | 1.2 |
| *3-phytase* | 500 (ftu/kg) |
| *Endoxylanase* | 10 (ftu/kg) |

| | |
|---|---|
| *Premix contains per kg of feed: vitamin A: 9615 IU/kg, vitamin D3: 2404 IU/kg, vitamin E: 38 mg/kg, Cu (Cu sulphate): 7 mg/kg, Fe (Fe sulphate): 33 mg/kg, I (Ca iodate): 2 mg/kg, Mg (Mg oxide): 71 mg/kg, Zn (zinc oxide): 53 mg/kg, Se (sodium selenite): 0.2 mg/kg, BHT: 96 mg/kg. | |

**Table 2: Nutrient composition of the experimental diet.**

| ***Nutrient*** | **Content** |
|---|---|
| | |
| *Dry matter, g*/*kg* | 841 |
| *Ash, g*/*kg* | 51 |
| *Crude protein, g*/*kg* | 190 |
| *Ether extract, g*/*kg* | 132 |
| *Crude fibre, g*/*kg* | 33 |
| *Nitrogen-free extract, g*/*kg* | 435 |
| *Metabolisable energy, MJ*/*kg* | 13.52 |
| *Methionine, g*/*kg* | 5.8 |
| *Lysine, g*/*kg* | 11.9 |
| *P, g*/*kg* | 4.6 |
| *Ca, g*/*kg* | 7.2 |
| *Na, g*/*kg* | 1.3 |

From the age of 24 days to the age of 48 days, the change in bodyweight was measured per bird, but pooled per pen as the experimental unit. At 42 days of age, one male bird per pen was euthanized by intravenous injection of sodium pentobarbital From the breast and tigh muscle, a sample was dissected and stored airtight at - 20°C until analysis. The breast muscle sample was taken at about one third from the distal end of the breast.

Muscle and tigh samples were analysed for their concentration of anserine, carnosine and taurine by high-performance liquid chromatography.

### Results

No animals died or became ill during the trial. Table 3 demonstrates that muscular anserine concentrations were overall higher than muscular carnosine concentrations. Both anserine and carnosine were higher in breast muscle than in thigh muscle, whereas taurine concentration was lower in breast muscle than in thigh muscle.

ß-alanine supplementation had substantially no effect on the muscular concentrations of carnosine, anserine and taurine, this in contrast to the findings of Tomonaga et al. (2005 and 2006) who used however much higher ß-alanine supplementations.

**Table 3: Effect of beta-alanine supplementation on histidine-containing dipeptides and taurine concentrations in thigh and breast of broiler chickens.**

| | **Thigh** | | **Breast** | |
|---|---|---|---|---|
| ***Beta-alanine, mg*/*kg*** | **0** | **500** | **0** | **500** |
| | | | | |
| | | | | |
| *Carnosine, mmol*/*kg* | 4.6 | 4.8 | 7.5 | 7.9 |
| *Anserine, mmol*/*kg* | 13.8 | 12.6 | 34.8 | 36.9 |
| *HCD, mmol*/*kg** | 18.3 | 17.4 | 42.3 | 44.8 |
| *Taurine, mmol*/*kg* | 5.8 | 5.6 | 2.0 | 2.2 |

| | | | | |
|---|---|---|---|---|
| **HCD= Histidine Containing Dipeptides* | | | | |

The birds in the ß-alanine group tended to have a higher bodyweight at slaughter, and indeed tended to grow faster.

**Table 8: Effect of beta-alanine supplementation on broiler chicken performance between 24 and 42 days of age.**

| ***Beta-alanine, mg*/*kg*** | **0** | **500** |
|---|---|---|
| *Initial bodyweight, kg* | 1.350 | 1.362 |
| *Final bodyweight, kg* | 2.810 | 2.874 |
| *Average daily gain, g* | 104 | 108 |

### Fish

### Materials and methods

A group of 24 carps were distributed over 12 aquaria with 2 fish each. All fish were previously housed in identical conditions, and fed the same diet as the control group in the trial. Every other aquarium was attributed to either a control diet or that control diet supplemented with 500 mg ß-alanine per kg. Feeding was done at 1.5% of the body weight, during 2 feeding times/day. To increase the level of oxidative stress, the carps were kept at 27°C (4°C above the advised temperature).

The change in bodyweight after the 14 day feeding experiment was measured per fish, but pooled per aquarium as the experimental unit.

### Results

**Table 9: Effect of beta-alanine supplementation on carp performance during 14 day experiment.**

| ***Beta-alanine, mg*/*kg*** | **0** | **500** |
|---|---|---|
| *Initial bodyweight, g* | 201.5 | 211.1 |
| *Final bodyweight, g* | 213.2 | 230.1 |
| *Average weight gain, g* | 11.8 | 19.0 |
| *Average feed intake, g* | 37.5 | 44.1 |
| *Feed conversion ratio, g:g* | 3.9 | 2.7 |

### References

Kalmar ID, Cools A, Buyse J, Roose P, Janssens GPJ, 2010. Dietary N,N-dimethylglycine supplementation improves nutrient digestibility and attenuates pulmonary hypertension syndrome in broilers pilot. Journal of Animal Physiology and Animal Nutrition 94: e339-e347.
Hu X., Hongtrakul K., Ji C., Ma Q., Guan S., Song C., Zhang Y., Zhao L. 2009. Effect of carnosine on growth performance, carcass characteristics, meat quality and oxidative stability in broiler chickens. Japan Poultry Science 46: 296-302.
Tomonaga S., Kaji Y., Tachibana T., Denbow M. D., Furuse M. 2005. Oral administration of ß-alanine modifies carnosine concentrations in the muscles and brains of chickens. Animal Science Journal 76: 249-254.
Jacob J .P., Blair R., Hart L.E. 1991. The effect of taurine transport antagonists on cardiac taurine concentration and the incidence of sudden death syndrome in male broiler chickens. Poultry Science 70 : 561-567.
Tomonaga S., Kaneko K., Kaji Y., Kido Y., Denbow M.D. 2006. Dietary ß-alanine enhances brain, but not muscle, carnosine and anserine concentrations in broilers. Animal Science Journal 77 : 79-86.
Mei L., Cromwell G.L., Crum A.D., Decker E.A. 1998. Influence of dietary ß-alanine and histidine on the oxidative stability of pork. Meat Science 49(1): 55-64.
Kim S., Takeuchi T., Yokoyama M., Murata Y. 2003. Effect of dietary supplementation with taurine, ß-alanine and GABA on the growth of juvenile and fingerling Japanese flounder Paralichthys olivaceus. Fisheries Science 69: 242-248.
Hebb C.D., Castell J.D., Anderson D.M., Batt J. 2003. Growth and feed conversion of juvenile winter flounder (Pleuronectes americanus) in relation to different protein-to-lipid levels in isocaloric diets. Aquaculture Volume:221, Issue: 1-4, Pages: 439-449.

## Claims

1. A method for the non-therapeutic treatment of animals selected from the group consisting of poultry, pigs and fish, which treatment comprises orally administering at least one ß-alanine compound to the animals in an amount of between 2 and 55 mmol/kg dry weight of said feed for the treatment of poultry and fish and between 2 and 25 mmol/kg dry weight of said feed for the treatment of pigs, which ß-alanine compound corresponds to the following formula (I): 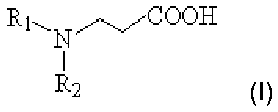 or to a salt or an amide thereof, the amide being of the following formula (II): 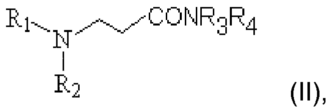 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

2. The method according to claim 1, wherein the ß-alanine compound is selected from the group consisting of ß-alanine, N,N-dimethyl ß-alanine, N,N-diethyl ß-alanine, N,N-di-n-propyl ß-alanine, N,N-diisopropyl ß-alanine, N,N-di-n-butyl ß-alanine, N,N-diisobutyl ß-alanine, N,N-di-tert-butyl ß-alanine, 3-acetamidopropanoic acid or mixtures or salts thereof, the ß-alanine compound being preferably ß-alanine or a salt thereof.

3. The method according to claim 1 or 2, wherein the ß-alanine compound is administered via said feed and/or via the drinking water of the animals.

4. The method according to any one of the claims 1 to 3, wherein the ß-alanine compound is administered in an amount of at least 5, preferably at least 10 and more preferably at least 15 mmol/kg dry weight of said feed.

5. The method according to any one of the claims 1 to 4, wherein the ß-alanine compound is administered in an amount of less than 50, preferably less than 40, more preferably less than 30, still more preferably less than 25 and most preferably less than 20 mmol/kg dry weight of said feed for the treatment of poultry and fish and in an amount of less than 22, preferably less than 20 and more preferably less than 17 mmol/kg dry weight of said feed for the treatment of pigs.

6. The method according to any one of the claims 1 to 5, wherein the ß-alanine compound is orally administered to poultry, in particular to poultry that are at least one week, preferably at least two weeks of age.

7. The method according to any one of the claims 1 to 6, wherein the ß-alanine compound is orally administered to said animals for the purpose of reducing the conversion ration of the feed used to feed the animals without lowering their bodyweight gain.

8. The method according to any one of the claims 1 to 6, wherein the ß-alanine compound is orally administered to said animals for the purpose of increasing their bodyweight gain.

9. Use of a ß-alanine compound for reducing the conversion ratio of feed used to feed animals selected from the group consisting of poultry, pigs and fish, without lowering their bodyweight gain, or for increasing the bodyweight gain of the animals, which ß-alanine compound is orally administered to said animals in an amount of between 2 and 55 mmol/kg dry weight of said feed for the treatment of poultry and fish and between 2 and 25 mmol/kg dry weight of said feed for the treatment of pigs, which ß-alanine compound corresponds to the following formula (I): 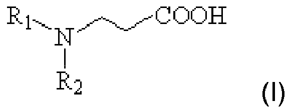 or to a salt or an amide thereof, the amide being of the following formula (II): 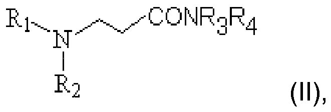 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

10. Use according to claim 9, wherein said ß-alanine compound is added to said animal feed.

11. A feed for poultry or for fish comprising between 2 and 55 mmol/kg dry weight of a ß-alanine compound, which ß-alanine compound corresponds to the following formula (I): 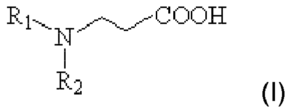 or to a salt or an amide thereof, the amide being of the following formula (II): 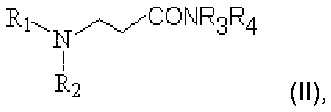 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

12. A feed according to claim 11, which comprises said ß-alanine compound in an amount of less than 50, preferably less than 40, more preferably less than 30, still more preferably less than 25 and most preferably less than 20 mmol/kg dry weight of said feed.

13. A feed for pigs comprising between 2 and 25 mmol/kg dry weight of a ß-alanine compound, which ß-alanine compound corresponds to the following formula (I): 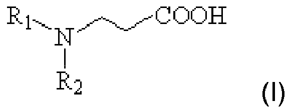 or to a salt or an amide thereof, the amide being of the following formula (II): 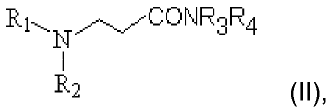 the R₁ and R₂ groups in formula's (I) and (II) being independently hydrogen, an acetyl or a straight or branched alkyl radical containing 1 to 4 carbon atoms, and the R₃ and R₄ groups in formula (II) being independently hydrogen or a straight or branched alkyl radical containing 1 to 4 carbon atoms.

14. A feed according to claim 13, which comprises said ß-alanine compound in an amount of less than 22, preferably less than 20 and more preferably less than 17 mmol/kg dry weight of said feed.

15. The feed according to any one of the claims 11 to 14, which comprises said ß-alanine compound in an amount of at least 5, preferably at least 10 and more preferably at least 15 mmol/kg dry weight of said feed.

16. The feed according to any one of the claims 11 to 15, wherein the ß-alanine compound is selected from the group consisting of ß-alanine, N,N-dimethyl ß-alanine, N,N-diethyl ß-alanine, N,N-di-n-propyl ß-alanine, N,N-diisopropyl ß-alanine, N,N-di-n-butyl ß-alanine, N,N-diisobutyl ß-alanine, N,N-di-tert-butyl ß-alanine, 3-acetamidopropanoic acid or mixtures or salts thereof, the ß-alanine compound being preferably ß-alanine or a salt thereof.
